Europäisches Patentamt

(19) European Patent Office

Office européen des brevets

(11) Publication number: **0 043 267**

**A1**

(12) # EUROPEAN PATENT APPLICATION

(21) Application number: **81302927.9**

(22) Date of filing: **29.06.81**

(51) Int. Cl.³: **C 10 G 21/00**
**C 10 G 21/28**

(30) Priority: **30.06.80 US 164039**

(43) Date of publication of application:
**06.01.82 Bulletin 82/1**

(84) Designated Contracting States:
**AT BE DE FR GB IT NL SE**

(71) Applicant: **UNION CARBIDE CORPORATION**
**270, Park Avenue**
**New York, N.Y. 10017(US)**

(72) Inventor: **Somekh, George Solomon**
**43 Winding Brook Road**
**New Rochelle New York, 10804(US)**

(72) Inventor: **Paulino, Forte**
**82 Homecrest Oval**
**Yonkers 10703 New York(US)**

(72) Inventor: **Vidueira, Jose Antonio**
**9-7 Granada Crescent**
**New York 10603 New YOrk(US)**

(74) Representative: **McCall, John Douglas et al,**
**W.P. THOMPSON & CO. Coopers Building Church Street**
**Liverpool L1 3AB(GB)**

(54) Method of separating aromatic and nonaromatic hydrocarbons in mixed hydrocarbon feeds.

(57) There is disclosed a process for separating aromatic and nonaromatic hydrocarbons which comprises a continuous solvent extraction-solvent decantation process. The process is particularly well suited for a feed comprising hydrocarbon mineral oils as the nonaromatic component · wherein the process by removing aromatics provides improved stability in said hydrocarbon mineral oils, normally susceptible to thermal degradation and oxidation and, further, decreases the tendency of the oil to form sludge. The process involves: (a) contacting the feed (10) in an extraction zone (16) with an aromatic selective solvent (18b) at a temperature of at least about 150°C to form an aromatic-rich solvent phase (24) containing primarily aromatic hydrocarbons and a raffinate (20) containing primarily nonaromatic hydrocarbons; (b) cooling (26,28) the aromatic-rich solvent phase (24) to bring about the formation of two phases; (c) introducing said cooled aromatic-rich solvent phase (24b) to a decantation zone (30) to provide an aromatic-rich extract (32) containing primarily aromatic hydrocarbons and a solvent phase (18) containing primarily aromatic selective solvent; (d) recycling said solvent phase (18b) to the extraction zone (16) of step (a), preferably, after solvent purification; and (e) recovering the aromatic-rich extract (32) of step (c) and the raffinate (21) of step (a).

FIG. 1

1.

DESCRIPTION

"METHOD OF SEPARATING AROMATIC AND NON-
AROMATIC HYDROCARBONS IN MIXED HYDRO-
CARBONS IN MIXED HYDROCARBON
FEEDS"

This invention relates to a process for the separation of aromatic and nonaromatic hydrocarbons from a mixed hydrocarbon feed, and more particularly, to the separation of aromatic and nonaromatic hydrocarbons in high yields from a mixed aromatic, naphthenic and paraffinic hydrocarbons feed while making efficient use of process components. Further, said process significantly decreases the energy requirements necessary for the separation of aromatic and nonaromatic hydrocarbons. The process is particularly well adapted for the separation of aromatic from naphthenic/paraffinic hydrocarbons in a mixed hydrocarbon feed wherein the nonaromatic component comprises mineral oils and is especially well-suited for lubricating oils (hereinafter lube oils).

The separation of aromatic and nonaromatic hydrocarbons (generally referred to as dearomatization) from mixed hydrocarbon feeds has long been recognized as necessary for a number of varied reasons. For example, when a BTX fraction (benzene, toluene, and xylene) is the aromatic fraction, it may be used as a raw material in the manufacture of petrochemicals, or as an additive for gasoline to increase its octane rating. Further, the nonaromatic fraction derived from these mixed feeds have varied uses as fuels, solvents, and the like and, thus, are also highly desirable. Such utilities for the aromatic and nonaromatic fractions have resulted in the development of varied dearomatization processes with the goal being to improve the economics of these processes.

2.

Of particular interest and difficulty is the purification of lube oil, wherein the separation of "aromatic" type hydrocarbons is necessary to improve the viscosity index, thermal and oxidation stability, and color of the lube oil. The presence of "aromatic" type hydrocarbons in lube oils affects the quality of these oils due to the low viscosity index, poor thermal and oxidation stability, high carbon residue, and poor color of such "aromatic" type hydrocarbons. The "aromatic" type hydrocarbons present in lube oils differ from the BTX fraction found in lighter hydrocarbon mixtures and as a result present different separation problems.

Various processes have been suggested for the separation of the aromatic and nonaromatic hydrocarbons of a mixed feed wherein the aromatic is a BTX fraction. Among these processes is a process employing an extraction column for separation of a BTX fraction which sends a glycol solvent/water solution, BTX and reflux to a two step distillation column. The resulting BTX is then distilled to remove water and entrained glycol. A similar process has been suggested wherein two distillation columns are employed. with BTX and water being distilled in the second column. In addition, a process using two distillation columns wherein the second column is employed to distill the BTX components, has been suggested.

These processes have not proven entirely satisfactory, when the separation of aromatic hydrocarbon involved other than a BTX fraction, e.g., the dearomatization of lubricating oils. Therefore, a number of processes have been proposed for the dearomatization of mixed hydrocarbon feeds containing various aromatic hydrocarbons. These processes have

3.

been directed in large part to the choice of the extraction solvent. For example, U. S. Patent Nos. 2,400,732 and 2,402,799 disclosed processes employing a solvent containing primarily water. Numerous water based solvents have been suggested for the extraction of aromatic hydrocarbons from mixed hydrocarbon feeds but to date they have proved not to be satisfactory. These include water based extraction solvents glycol/water wherein up to 50 percent glycol is added (U.S. Patent 2,400,802); methanol/ water (U.S. Patent 3,985,644); water/non-bxygenated organic solvents (U.S. Patent 2,298,791); water/amines (U.S. Patent 2,401,852); and water/inorganic salts, acid or bases, or organic substances) (U.S. Patent 2,403,485). The problems associated with employing a water-based extraction solvents are well-known in the prior art.

To help overcome the problems associated with employing water based extraction solvents various processes have been suggested. For example, U.S. Patent 1,783,203 discloses the use of dry alcohols ($C_1$-$C_3$) for treating heavy petroleum oils. The problems relating to the flammability and toxicity of such alcohols are well-known in the art. U.S. Patent 1,908,018 discloses the use of glycol ethers in a process for refining mineral oils by separating paraffinic and naphthenic portion thereof wherein the glycol ether is mixed with the mineral oil and the mixture is cooled to give a two-layer system. With a paraffinic layer and a naphthenic layer. U.S. Patent 2,337,732 discloses the use of ethanolamines for removing aromatics from a hydrocarbon distillate, comprising gasolines or light hydrocarbons ($C_1$-$C_5$), by an extraction-distillation process. U.S. Patent 2,295,612 discloses the use of low molecular weight polyhydric alcohols for separating aromatic mixtures to obtain resin forming

4.

compounds. U.S. Patent 2,129,282 discloses the use of a beta,beta' dichlorodiethyl ether and 2-30% propylene glycol solvent for extracting naphthenic impurities from lubricating oils at temperatures from 120°F to 200°F. U.S. Patent 3,379,788 discloses the use of alkylene oxide adducts of phenyl glycidyl ether and U.S. Patent 2,834,820 discloses the use of mixed alkylene oxide adducts of ethylene or propylene oxide as solvents in dearomatization processes.

To overcome the relatively low yields, purities and solvent recovery problems of the above processes several dearomatization processes have been suggested employing extraction and distillation. These include: solvent extraction-steam distillation processes (such as those disclosed in U.S. Patent Nos. 3,417,033; 3,714,034; 3,779,904; 3,788,980; 3,755,154 and 3,966,589); processes employing multiple extraction zones and azeotropic distillation (e.g. U.S. Patent 3,789,077); processes employing distillation and stripping columns (U.S. Patent Nos. 4,048,062 and 4,177,137); and multiple distillation processes (e.g. U.S. Patent 3,461,066).

Unfortunately, these processes employ distillations. Further, high capital and energy costs are generally associated with employing such processes; therefore, processes have been sought whereby these problems may be minimized. These include:

U.S Patent No. 3,431,199 discloses a method of separating aromatic hydrocarbons from a mixed hydrocarbon feed by use of solvents comprising diethylene glycol, dipropylene glycol, sulfolane and mixtures thereof. The process is directed to the separation of light aromatics by extraction at temperatures preferably between 80°C. and 130°C. and employs azeotropic distillation with acetone to effect separation of the aromatic hydrocarbons. The process preferably employs solvent with 2% to 8% by weight water.

5.

U.S. Patent No. 3,551,321 discloses an extraction distillation process which employs a sulfolane-type solvent.

U.S. Patent No. 3,985,644 discloses a method of separating naphtha into aromatic and paraffin-rich fractions by solvent extraction with a methanol-water mixture. The solvent is separated from the aromatic-rich phase by lowering the temperature of the mixture. As indicated therein, the solvent comprises methanol/water mixtures. These are highly toxic and flammable mixtures.

U.S. Patent No. 4,086,159 discloses a method for separating aromatic hydrocarbons from mixed hydrocarbon feeds by use of an ethoxylated alkane polyol solvent in an extraction-distillation process. The ethoxylated alkane polyol solvents high boiling point provides for the recovery of higher boiling aromatics such as ethylbenzene and poly-substituted benzenes. The process necessarily requires sizable quantities of energy to carry out the energy intensive distillation steps.

U.S. Patent No. 4,179,362 discloses a method for separating aromatic-containing petroleum fractions into aromatic-rich and paraffinic-rich hydrocarbon streams by use of a methanol/water extraction solvent (at least 10 volume percent water) in an extraction zone at a temperature of about 150°-450°F. The extraction step is followed by additions of water to the aromatic-rich extract such that the water/methanol solvent contains at least 80% water, by volume. The use of methanol/water solvents for treating higher distillates tends to require higher process pressures and suffers from the safety constraints associated with methanol/water solvents, e.g. high flammability and high toxicity.

6.

The above processes show the intense interest in developing a dearomatization process which lowers the cost of those processes heretofore used commercially. U.S. Patent 3,985,644 suggests one such method for achieving this goal, i.e. by reducing the use of energy-intensive steps, e.g. distillation.

Such dearomatization processes are of particular interest in the dearomatization of mineral oils, e.g. lube oils. Dearomatized lubricating oils are, generally speaking, naphthenic-paraffinic type viscous materials having low rate of viscosity change with change in temperature, i.e., relatively high viscosity index, a high degree of thermal and oxidation stability, low carbon-forming tendency, good color, and high flash points. Lubricating oil feedstocks are generally recovered as heavy distillates or bottoms from the vacuum distillation of crude oils. A crude lube oil fraction contains many different chemical components, e.g., paraffins, naphthenes, aromatics, and the like. In order to obtain refined lubricating oils of relatively good quality and high viscosity index, the practice has been to remove components, such as aromatic and polyaromatic compounds, which tend to lower the viscosity index of the oil. The removal of these aromatic components has heretofore been carried out by processes as above-described and processes such as disclosed in U.S. Patent Nos. 2,079,885; 2,342,205 and 3,600,302.

U.S. Patent 2,079,885 discloses a process for refining hydrocarbon oils containing aromatic and nonaromatic components by counter current extraction .

- with selective solvents such as furfural or phenol,

7.

cooling the aromatic-rich extract and oiling out raffinate and recycling the oiled out raffinate.. Unfortunately, such a process results in some raffinate loss due to dissolving in the extract.

U.S. Patent 2,342,205 discloses a solvent recovery scheme wherein aliphatic and aromatic hydrocarbons are washed with water and then distilled.

U.S. 3,600,302 discloses a method of upgrading petroleum distillate fractions by extraction with a mixed solvent comprising an aromatic organic compound having a 6-membered ring containing at least one polar functional group and a diethylene glycol ether having the general formula:

$$R-(OCH_2CH_2)_2-OH$$

wherein R is lower alkyl.

There is disclosed in copending application Attorney's Docket Number 12805 (U.S. Applications Serial Nos. 164228 and 267427 and European Application No. case A2363/U). A solvent extraction-solvent decantation process wherein an anti-solvent is employed in the decantation zone to improve process efficiency.

The process of this invention is to be distinguished from the prior art in that the process provides a solvent extraction-solvent decantation process that is economically advantageous, i.e. energy efficient, and overcomes problems inherent in the above described prior art processes.

This invention provides a process for the separation of aromatic and nonaromatic hydrocarbons from a mixed hydrocarbon feed in which an aromatic selective solvent, preferably relatively low molecular weight polyalkylene glycols and mixtures thereof, is utilised in an extraction-

8.

decantation process whereby aromatic and nonaromatic hydrocarbons are recovered without employing distillations to recover aromatic selective solvents and products. The aromatic and nonaromatic products are recovered in relatively high yield using a minimum of process equipment, thereby minimizing capital investment.

According to the present invention a process is provided for the dearomatization of a mixed hydrocarbon feed which process comprises the following steps:

(a) contacting the feed in an extraction zone with an aromatic selective solvent at a temperature of at least about 150°C to provide an aromatic-rich solvent phase containing primarily aromatic hydrocarbons and a raffinate containing primarily nonaromatic hydrocarbons;

(b) cooling the aromatic-rich solvent phase to bring about the formation of two phases;

(c) introducing said cooled aromatic-rich solvent phase to a decantation zone to provide an aromatic-rich extract containing primarily aromatic hydrocarbons and a solvent phase containing primarily aromatic-selective solvent;

(d) recycling said solvent phase to the extraction zone of step (a); and

(e) recovering the aromatic-rich extract of step (c) and the raffinate of step (a).

Further, it has been discovered that increased purity and yield may be achieved by employing additional extraction steps. These additional extraction steps comprise contacting the solvent, prior to recycling, with the mixed hydrocarbon feed or with raffinate from step (a). In addition, a process for extracting the small amounts of solvent present in the products of step (e) is disclosed.

9.

As noted above, there is an industrial need for an energy efficient process for the separation of aromatic and nonaromatic hydrocarbons in a mixed hydrocarbon feed, particularly in the dearomatization of crude lube oils. Naphthas, heating oils, light oils, cracked gasolines, dripolenes, lubricating oils, kerosene and the like, can contain between 20 to 90 percent by weight aromatic-type hydrocarbons, e.g. BTX or polyaromatics. Since the individual hydrocarbon compounds which make up these hydrocarbon feed streams are well known, they will not be discussed extensively, except to note that the mixed hydrocarbon feed employed herein may be any petroleum hydrocarbon fraction containing aromatics, such as for example naphthas (virgin or cracked) kerosene, gasoline, heating oils, lubricating oils and residual oils. Preferably, the feed stream is a heavy distillate or a lube oil fraction.

The aromatic hydrocarbons present in heavy hydrocarbon feeds, e.g. lubricating oils, generally include alkylbenzenes, indanes, tetralins, indenes, naphthalenes, fluorenes, acetonaphthalenes, biphenyls, phenanltrenes, anthracenses, diacenaphthalenes, pyrenes chripenes, diacetoanthracenes, benzopyrenes and other various aromatic feed components.

The solvents which are employed in the instant process are varied. Typical desirable characteristics for solvents of the subject process may generally be stated to be as follows: a) high selectivity for the aromatic feed components at the high extraction temperature; b) high solvent capacity for the aromatic feed components at the higher extraction temperatues (i.e., low solvent to feed ratios); c) low

0043267

10.

vapor pressure at the high temperature of extraction to avoid the use of pressurized equipment; d) low capacity for the aromatic feed components at the lower decantation temperature; e) chemical and thermal stability under the process conditions; f) adaptable to a wide range of feeds; g) available at a reasonable cost; h) noncorrosive to conventional metals of construction; i) relatively low toxicity, i.e. environmentally safe; and j) adequately high density difference between the solvent and oil.

The solvents used in the instant process tend to be water-miscible organic liquids (at process temperatures) having a boiling point and decomposition temperature higher than the extraction temperature. The term "water miscible" solvents includes those solvents which are completely miscible with water over a wide range of temperatures and those solvents which have a high partial miscibility with water at room temperature, since the latter are usually completely miscible at process temperatures. The solvents are generally polar and contain carbon, hydrogen and oxygen, with some exceptions.

Representative of the solvents employed in this process are the low molecular weight polyalkylene glycols and the like. Examples of the solvents employed are ethylene glycol, diethylene glycol, triethylene glycol, tetraethylene glycol, 1,3-propylene glycol, 1,4 butylene glycol, 1,3-butylene glycol, 1,2-butylene glycol, 1,5-pentaethylene glycol, mixtures thereof and the like. The preferred solvents are diethylene glycol, triethylene glycol, tetraethylene glycol, or mixtures thereof.

It is believed that other solvents having the aforementioned solvent characteristics may be employed

11,

in addition to aforementioned solvents, among these being: amines such as diethylene triamine and triethylenetetramine; alkanolamines such as monoethanolamine, diethanolamine, and triethanolamine; sulfolane; N-methyl-2-pyrrolidone; aromatic compounds such as dimethylphthalate and diphenyl ether; and halogenated compounds such as perchloroethylene, 1,1,2- trichloroethylene and 1,2,3-trichloropropane, or mixtures thereof.

Well-known anti-solvents such as ethylene glycol, glycerine and the like may be employed in the extraction zone in conjunction with the aromatic-selective solvent to improve the selectivity of the solvent for said aromatics.

Generally to accomplish the extraction, the ratio of solvent to hydrocarbon feed in the primary extractor (as hereinafter discussed) is in the range from about 2 to about 20 parts by weight of solvent to one part by weight of feed the ratio 3 to 1 to 15 to 1 being preferred and the ratio 4 to 1 to 12 to 1 being the most preferred. The broad range may be expanded upon depending on the solvent or feedstock employed. The optimum solvent to feed ratios and temperature employed herein depend upon whether high recovery (yield) or high purity (quality) is being emphasized, although the instant process will generally result in both good recovery and purity.

The instant process is further characterized in that the pressures employed in the extraction and the decantation steps are generally less than about 100 psig. This is highly advantageous in terms of ease of operation and the capital expenditure required for carrying out the separation process. The actual

12.

pressures in the extraction zone depend on the particular hydrocarbon feed treated, the solvent employed and the selected temperature at which the extraction is carried out.

The temperature in the extraction zone is generally in the range of from about 150°C to about 250°C and is preferably in the range of from about 200°C to about 250°C with 225°C ± 15°C being the most preferred temperature range. The decantation zone is generally maintained at a temperature of from about 50°C to about 125°C with from about 75°C to about 100°C being preferred. The temperature employed in the decantation zone depends in part upon solubility of the aromatic hydrocarbon in the extraction solvent and the viscosity of the selected solvent at the settling temperature.

The equipment used in the instant process, both for the extraction and the decantation, is of conventional design, e.g., an extraction column of the multistage reciprocating type containing a plurality of perforated plates centrally mounted on a vertical shaft driven by a motor in an oscillatory manner can be used as well as columns containing pumps with settling zones, and sieve trays with upcomers.
(Counter-current flow is generally utilized in the extraction columns.) The decantation can be conducted in a tank with no internal elements, perferably the decantation tank contains coalescing elements to enhance phase separation in the decantation tank.

Heat exchangers, reservoirs, and solvent regenerators are also of conventional design as well as are the various extractors and decanters used, in the

13.

various embodiments hereinafter described. The extractors employed for other than the primary extraction can be multistage extractors or any type suitable for said extraction processes.

This process provides for an overall recovery of the aromatic hydrocarbon of from about 35 to about 95 percent or better, based upon the weight of aromatic in the original hydrocarbon feed, and is usually in the range of about 50 to about 65 percent by weight. Additional processing steps, as hereinafter described, may be employed to obtain recoveries of about 95 percent by weight. Overall recovery of the nonaromatic hydrocarbon is about the same.

The invention will now be described by way of example mainly by reference to the accompanying drawings, in which:-

FIG. 1. is a flow sheet depicting one embodiment of this invention to illustrate how the extraction/decantation process of this invention may be employed.

FIG. 2. is a flow sheet depicting a second embodiment of this invention, according to FIG. 1., wherein, in addition, a solvent back extraction with mixed hydrocarbon feed is employed.

FIG. 3. is a flow sheet depicting a third embodiment of this invention to illustrate the process depicted in FIG. 1., wherein, in addition, a solvent back extraction with raffinate is employed.

FIG. 4. is a flow sheet depicting a water-wash system employed in conjunction with the processes illustrated in FIGS 1, 2 and 3 and 4 to provide for recovery of aromatic selective solvent from the raffinate and aromatic-rich extract phase.

14.

The process involves in the first instance, the extraction of a hydrocarbon feed with an aromatic selective solvent at a pressure generally less than about 100 psig. and at a temperature of at least about 150°C and preferably in the range of about 150°C and about 250°C, followed by cooling and decantation, wherein a solvent phase is separated from an aromatic-rich extract phase at a temperature from about 50°C to about 125°C, preferably at about 75°C to about 100°C.

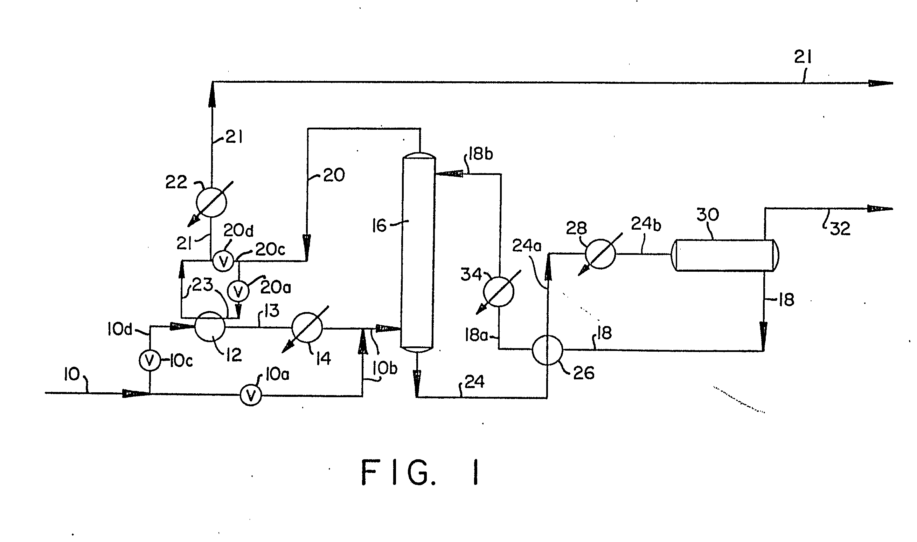

Referring to Fig. 1:

Hydrocarbon feed introduced via line 10, if the feed is hot, passes through valve 10a and line 10b before introduction near the bottom of extraction column 16 ("primary" extraction step). If the feed is cold, valve 10a is closed and valve 10c is opened so that feed passes through line 10d.to heat exchanger 12 where it is heat-exchanged with raffinate. The feed then passes via line 13 to heater 14 wherein the feed is heated and is introduced near the bottom of extraction column 16 (primary extraction column). It is to be understood that introduction of the feed near the bottom of extraction column 16 is related to the density of the feed and that if density of the feed is higher than the density of the solvent that the feed would be introduced near the top of extraction column 16. Solvent having a temperature in the range of about 150°C to about 250°C enters ex-

15.

traction column 16 near the top of extraction column 16 via line 18 b and percolates down the column removing aromatic hydrocarbons from the feed as the feed percolates upwards.

Raffinate containing primarily nonaromatic feed components exits extraction column 16 via line 20 and passes through opened valve 20a, to heat exchanger 12 where the raffinate may be cooled by employing it to preheat the mixed hydrocarbon feed. Raffinate passes to heat exchanger 22 via line 21 where it is further cooled. The raffinate exits the process through line 21.

An aromatic-rich solvent phase, containing primarily aromatic hydrocarbons and solvent, exits extraction column 16 via line 24 and passes to heat exchanger 26 where it is cooled with the solvent phase from decantation tank 30 and further cooled in cooler 28 (depicted as a cold water cooler).

The aromatic-rich solvent phase is introduced to decantation tank 30 where it separates into an aromatic-rich extract phase, and a solvent phase, said solvent phase containing primarily aromatic selective solvent. The solvent phase exits decantation tank 30 via line 18, is heate in heat exchanger 26 and heater 34 (shown using steam) and passes through line 18b to near the top of extraction

16.

column 16 as recycled extraction solvent. The aromatic-rich extract exits the decantation tank 30 via line 32.

The aforementioned continuous process provides for an overall recovery for aromatic and nonaromatic hydrocarbons in the range of about 25 percent to about 85 percent by weight and generally is in the range of from about 50 to about 65 percent by weight.

## BACK EXTRACTION WITH MIXED FEED

The above-described process is highly advantageous in separating aromatic and nonaromatic hydrocarbons. It may often be desirable to separate a greater portion of the aromatic component from the mixed feed, i.e., it may be desirable to remove over 85 percent by weight of the aromatic hydrocarbon from the mixed hydrocarbon feed. It has been found that this may be achieved by the addition of a second extraction step, i.e. secondary extraction, wherein the small quantities of aromatic hydrocarbons in the relatively cool solvent phase from the decantation tank are back extracted with a portion of the mixed hydrocarbon feed (hereinafter "primary feed"). The secondary extraction removes aromatic hydrocarbons from the solvent phase prior to introduction of the solvent phase to the primary extraction step. The removal of aromatic hydrocarbons provides for increased yield and purity of the nonaromatic and aromatic hydrocarbons in the raffinate and in the aromatic-rich extract phase, respectively.

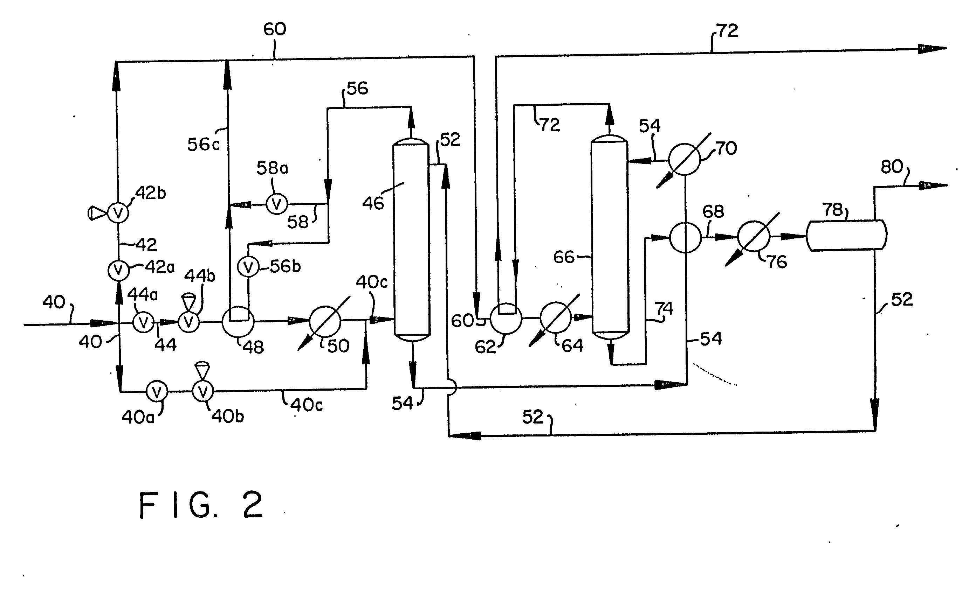

Referring to Figure 2:

Figure 2 shows a process as above-discussed and as depicted in Figure 1 except that extraction column 46 has been added to provide for the extraction of the

17.

solvent from the decantation tank with relatively cool primary feed, e.g., feed at about 100°C.

The process parameters, i.e., temperature, pressure, solvent selection and the like, are as hereinabove discussed except as to secondary extraction column 46, discussed hereinafter.

Primary feed introduced through line 40 is split into two streams. One stream passes directly to extraction column 66 via lines 42 and 60 where it undergoes extraction with aromatic selective solvent, as hereinbefore described.

If the incoming mixed hydrocarbon feed is relatively cool, e.g., about 100°C, valves 40a and 42a are opened and valve 44a is closed while valves 40b and 42b are adjusted to split the incoming feed. The amount of feed passing through lines 40 and 42 may be adjusted as required but the ratio of feed in either of said lines to the feed in the other of said lines is generally in the range of from about 3 to 1 to about 1 to 3.

If the feed is hot, e.g., above about 125°C, that portion of the stream going to extraction column 46 (secondary extraction zone) must be cooled, e.g., to a temperature of about 100°C. (The exact temperature being dependent on the solvent selection.) The feed is cooled by closing valve 40a and opening valve 44a such that the feed passes via line 44, through control valve 44b, to heat exchanger 48 and cooler 50 (shown as a cold water cooler). The cooled feed is then introduced near the bottom of extraction column 46 via line 40c, i.e. the secondary extraction column, wherein the solvent phase from decantation tank 78 is back extracted with relatively cool primary feed.

18.

The solvent phase from decantation tank 78 is introduced to extraction column 46 through line 52 and percolates down extraction column 46, as feed percolates upward, although this order depends on feed density, as hereinbefore discussed. Solvent exits extraction column 46 depleted of some portion of the aromatic hydrocarbons previously contained therein, i.e., the solvent in line 54 has a lower weight percent of aromatic hydrocarbons than the solvent in line 52. The feed leaving extraction column 46 via line 56 (hereinafter "secondary feed") contains a higher concentration of aromatic components than does the feed in line 40c.

The secondary feed, i.e., feed having an increased concentration of aromatic components, may be heat exchanged, if necessary, to raise its temperature prior to addition to the remainder of the primary feed from line 42, by opening valves 56b and 44a, closing valves 40a and 58a, whereby the secondary feed is heat exchanged in heat exchanger 48 with primary feed in line 44 and passes through line 56c into line 60. If, instead, valves 40a and 58a are open and valves 56b and 44a are closed the secondary feed passes directly to line 60 to form the combined (primary and secondary feeds) feed for introduction to primary extraction column 66.

The combined feed is passed via line 60 through heat exchanger 62 and heater 64 (shown as a steam heater) wherein the feed is heated to a temperature of from about 150°C to about 250°C, e.g., preferably about 200°C when the solvent selected is diethylene glycol. The heated feed is then introduced near the bottom of

19.

extraction column 66 (primary extraction zone) while solvent from the bottom of extraction column 46 passes via line 54 to heat exchanger 68 and heater 70 wherein the solvent phase is heated to at least 150°C and preferably from about 150°C to about 250°C. The heated solvent phase is introduced near the top of extraction column 66. The solvent phase percolates down extraction column 66 removing aromatic hydrocarbons from the combined feed as the feed percolates up the extraction column.

Alternatively, the higher concentration of aromatic hydrocarbons in the secondary feed of line 56 and 56b, as compared to the concentration of aromatic hydrocarbons in the primary feed in line 42, may be beneficially employed by introducing the secondary feed in line 56 at a point somewhat lower in primary extraction column 66 than is the primary feed in line 42. In this case an additional heat exchanger and heater would be employed (not shown) to heat the secondary feed stream prior to being introduced to extraction column 66.

The raffinate, now essentially depleted of aromatic hydrocarbons, exits the top of extraction column 66, passes through heat exchanger 62 and leaves the process via line 72. The raffinate comprises about 70 to about 90 percent nonaromatic hydrocarbons, about 10 to about 30 percent by weight aromatic hydrocarbons and about 1 percent to about 3 weight percent dissolved and entrained solvent. (This solvent may be recovered by a water wash process as discussed hereinafter).

0043267

20.

The aromatic-rich phase from extraction column 66 containing primarily aromatic hydrocarbons, solvent and small amounts of nonaromatic hydrocarbons, leaves the bottom of the extraction column 66 via line 74 and undergoes cooling in heat exchanger 68 and cooler 76. The aromatic-rich solvent phase, enters decantation tank 78 where the separation into two phases occurs, i.e. an aromatic-rich extract phase and a solvent phase. The aromatic-rich extract phase exits the process via line 80 and the solvent phase is recycled to extraction column 46 via line 52.

The overall recovery of aromatic and nonaromatic hydrocarbons is in the range of from about 60 to about 90 percent by weight, and generally in the range of from about 70 percent to about 85 percent by weight.

The solvent to feed ratios employed for the primary extraction are similar to those described hereinbefore with reference to FIG. 1. The solvent to feed ratios employed for the secondary extraction are generally in the range of about 5 to about 25 parts solvent per 1 part feed by weight, with about 7 to about 22 being preferred and about 10 to about 18 being the most preferred.

The temperatures employed in extraction column 66 and decantation tank 78 are as hereinbefore discussed for Fig. 1.

The use of the relatively cold primary feed to extract aromatic hydrocarbons from the solvent phase of

21.

the decantation zone prior to reintroduction of the solvent phase to the primary extraction zone provides solvent sufficiently low in aromatic hydrocarbons so as to generally provide for removal of between about 70 percent to about 80 percent by weight of the aromatic hydrocarbon from the feed with up to 90 percent removal possible.

## BACK EXTRACTION WITH RAFFINATE

As above-discussed, the process of Figure 2 is advantageous in achieving removal of aromatic hydrocarbon from the solvent phase prior to recycling to the primary extraction zone such that up to about 90 percent by weight of the aromatic and nonaromatic hydrocarbons . are recoverd. These recoveries can be improved even further if, instead of carrying out the back extraction with cooled primary feed, the back extraction is carried out using part of the primary raffinate product, i.e. raffinate from the primary extraction. By using the raffinate product as the extractant for the solvent phase of the decantation zone it has been found that it is possible to recover greater than about 90 percent by weight of the aromatic and nonaromatic hydrocarbons.

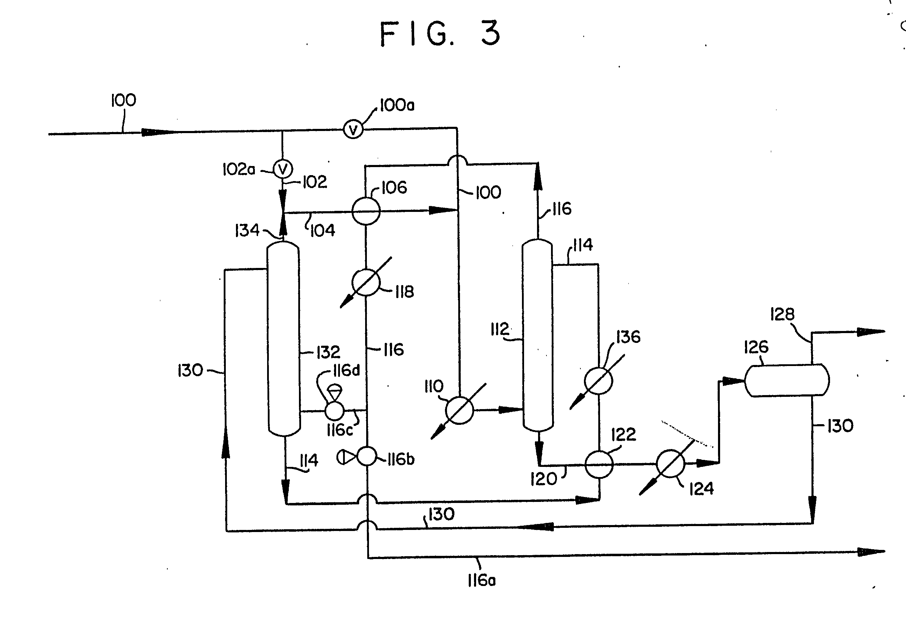

Referring to Figure 3:

Figure 3 shows an embodiment of the invention wherein small amounts of aromatics in the solvent phase from the decantation tank are extracted with part of the raffinate from the primary extraction step.

The process parameters, i.e., temperature, pressure, solvent selection and the like are as hereinbefore described, except as hereinafter noted.

Primary feed is introduced through line 100. If the feed is relatively cool (i.e., about 100°C), valve 102a is opened and valve 100a is closed so that the feed passes through line 102 wherein it is mixed with secondary raffinate in line 104. (Secondary raffinate is raffinate product from the primary extraction zone after having been employed to extract aromatic hydrocarbons from the solvent phase obtained from the decantation tank.) The combined feed (primary feed and the secondary raffinate of line 134) is preheated in line 104 by heat exchanging with primary raffinate product in heat exchanger 106. The combined feed passes through line 100 to heater 110 (shown as a steam heater) wherein the feed is heated to from about 150°C to about 250°C before introduction near the bottom of extraction column 112 (the primary extraction zone).

Alternatively, if the feed was relatively hot (i.e., about 150°C) valve 100a is opened and valve 102a is closed such that the incoming feed combines with the secondary raffinate in line 100 prior to entering extraction column 112. It is obvious to those skilled in the art that other heat exchange schemes may be employed depending on the temperature of the incoming feed.

Similarly as discussed for the process of Figure 2, the secondary raffinate product may be introduced to extraction column 112 separately if the concentration of the aromatic component therein is

·23.

significantly different from that of the incoming feed. In such a case the stream with the higher concentration of aromatic components is preferably introduced into the primary extraction column at a point near the bottom extraction column 112.

Solvent is introduced to extraction column 112 at about 150°C to about 250°C, preferably 200°C, via line 114 and percolates down the column as the combined feed percolates up the column. Raffinate product (primary raffinate) exits extraction column 112 via line 116 and is cooled (preferably to about 100°C for diethylene glycol by successive cooling in heat exchanger 106 and cooler 118 (shown as a cold water cooler). The aromatic-rich solvent phase exits extraction column 112 via line 120, is cooled by heat exchange with cool solvent from extraction column 132 in heat exchanger 122 and is further cooled (preferably to about 100°C) in heat exchanger 124 (shown as a cold water heat exchanger).

The cooled aromatic-rich solvent phase from extraction column 112 is introduced to decantation tank 126 wherein the solvent fraction separates into an aromatic-rich extract phase and a solvent phase. The aromatic-rich extract phase, generally having a density relatively lower than the solvent phase, exits decantation tank 126 and exits the process via line 128.

The solvent phase in decantation tank 126 passes through line 130 to extraction column 132 (secondary extraction) for extraction of aromatic hydrocarbons remaining in the solvent phase. The

extraction with primary raffinate lowers the concentration of aromatic hydrocarbons in the solvent phase. The solvent phase is introduced near the top of extraction column 132 and primary raffinate (obtained by splitting a portion of the primary raffinate in line 116 between lines 116a and 116c is introduce near the bottom of extraction column 132). The primary raffinate introduced near the bottom of extraction column 132 percolates up the column, removing aromatic hydrocarbons from the solvent phase. Secondary raffinate leaves column 132 via lines 134 and 104 and is recycled to the primary extractor column, i.e., extraction column 112. The solvent phase leaving extraction column 132 via line 114 (having a lower concentration of aromatic feed components than the solvent in line 130) is introduced via line 114 to extraction column 112 after being preheated with the aromatic-rich solvent phase in heat exchanger 122 and heater 136 (shown as steam heater).

The extraction columns employed herein preferably have at least about three theoretical stages and preferably no more than about twelve with about 4 to 6 theoretical stages being preferred.

The solvent to feed ratios (combined feed) used in extraction column 112 (primary extraction) may be in the range of about 2 to about 12 parts by weight solvent to one part by weight combined feed with about 4 to about 8 being preferred. The ratio of solvent to primary raffinate in extraction column 132 (secondary extraction) is in the range from about 5 to about 35 parts by weight solvent to 1 part primary raffinate with about 10 to about 20 being preferred.

25.

Extraction column 112 is preferably operated at a temperature of about 150°C to about 250°C, as hereinbefore discussed, and extraction column 132 and decantation tank 126 are operated at a temperature of from about 50°C to about 125°C.

## SOLVENT RECOVERY

In each of the above embodiments the aromatic-rich extract phase and raffinate contain minor amounts of aromatic selective solvent. Typically the recovery of entrained and dissolved solvent has been carried out by the use of distillation processes or by using water as a stripping medium in a hydrocarbon-solvent distillation column.

It has been discovered that the use of distillation processes to recover the extraction solvent can be eliminated by employing a two step extraction process. In the first step aromatic selective solvent is extracted from the aromatic-rich extract and raffinate with water. In the second step the solvent is extracted from the water with a relatively hot feed, i.e., feed at a temperature of from about 125°C to about 225°C with about 175°C to about 200°C being preferred.

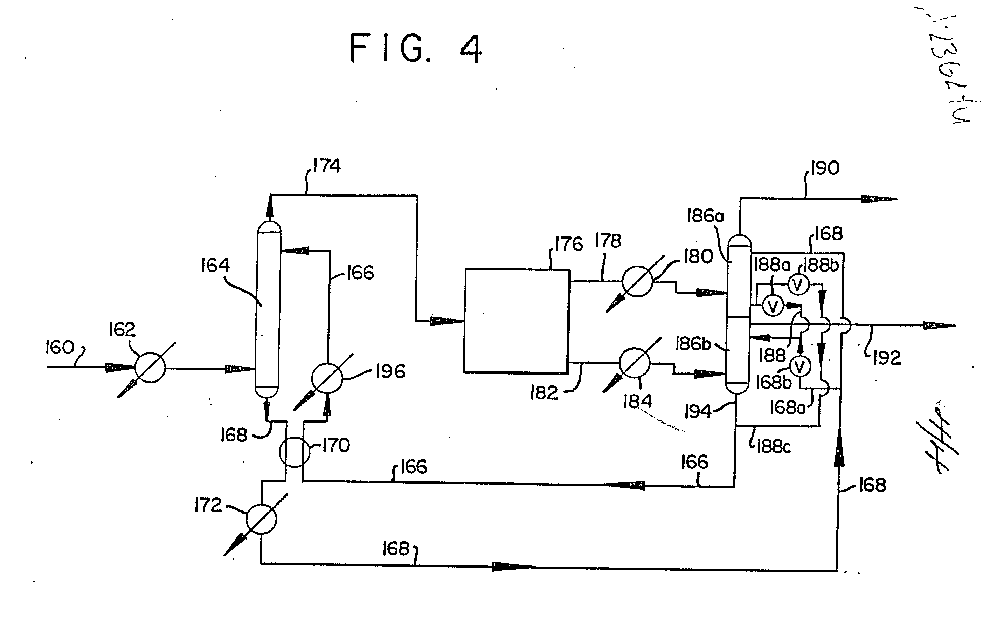

Referring to Figure 4:

Figure 4 depicts a solvent recovery system wherein a mixed hydrocarbon feed introduced via line 160 is heated by heater 162 to a temperature of about 125°C to about 225°C prior to introduction near the bottom of extraction column 164. The aqueous stream, derived from washing the aromatic-rich extract phase and raffinate of dearomatization process 176 (e.g., as depicted in FIGS. 1,2 or 3), containing primarily

26.

water and solvent is introduced via line 166 near the top of extraction column 164. The aqueous stream containing primarily water, leaves extraction column 164 via line 168 and the mixed hydrocarbon feed leaves via line 174 and enters a dearomatization process 176, as hereinbefore described. The product of process 176 having the lower concentration of aromatic hydrocarbons leaves process 176 via line 178 and the product having the higher concentration of aromatic hydrocarbons leaves process 176 via line 182. The product in line 178 and 182 are cooled by heat exchangers 180 and 184, respectively, preferably to a temperature of about 75°C to about 100°C.

The product in line 178 is introduced near the bottom of extraction column 186a and is contacted with water introduced via line 168 such that the stream leaving via line 190 is essentially free of extraction solvent.

The product in line 182 is introduced near the bottom of extraction column 186b via line 182 wherein it is contacted countercurrently with aqueous solution from line 188 or alternatively with water from line 168.

The aqueous stream in line 168 is employed to wash the products in lines 178 and 182 and is introduced to extractors (water wash) 186a and 186b by opening valve 168b. Water, containing solvent, exits solvent extractor 186a via 188c by closing valve 188a and opening valve 188b. Water and solvent from extractor 186b, in line 194, and water and solvent from extractor 186a, in line 188c, join to form the water phase for extraction with mixed hydrocarbon feed. Alternatively, valve 188b is closed and valve 188a is open and the water introduced to solvent extractor 186a is introduced to column 186b via line 188.

27.

The stream exiting extraction column 186b via line 192 is essentially free of extraction solvent. The combined aqueous stream in line 166 passes through exchangers 170 and steam heater 196 prior to introduction to extraction column 164. Stream 168 is cooled in heat exchanger 170 and water cooled in heat exchanger 172.

### EXAMPLE

As above-noted, the instant process is well suited for a variety of mixed hydrocarbon feeds and is particularly well-suited for the dearomatization of hydrocarbon feeds comprising lubricating oils.

As above noted, lubricating oils are, generally speaking, paraffinic or naphthenic type materials having low rate of viscosity change with change in temperature, i.e., high viscosity index.

The embodiment of Figs. 3 and 4 are employed to remove the aromatic feed components from a lubricating oil feed comprising about 30 percent by weight of mixed butyl napthalenes (i.e., mono-, di-, and tri-butyl) and 70 percent by weight paraffinic hydrocarbons, i.e., lubricating oil.

The solvent employed in the instant example is diethylene glycol. The temperature of the primary extraction column is about 200°C.± 10°C. The vapor pressure at the top of the extraction zone is about 14 psig.

After primary extraction of the combined feed the aromatic-rich solvent phase is introduced to the decantation zone after being cooled to about 80°C. The back extraction of solvent is achieved by use of primary raffinate as the extractant, i.e., the embodiment of the process shown in FIG. 3 is employed. The solvent phase from the decantation zone is extracted with about 50 percent of the primary raffinate, said primary raffinate. being at a temperature of about 80°C.

The recovery and purity of the aromatic and nonaromatic hydrocarbons is shown in Table I for various solvent to feed ratios (solvent to feed ratios of the primary extraction zone.)

## TABLE I

| Solvent to Feed Ratio (wt./wt.) | Number of Theoretical Stages in Primary Extractor | Recoveries* (wt. percent of primary feed) | | Purity** | |
|---|---|---|---|---|---|
| | | Nonaromatics | Aromatics | Nonaromatics in Nonaromatic Product | Aromatics in Aromatic Product |
| 12:1 | 4 | 95 | 89 | 95.3 | 88.4 |
| 12:1 | 6 | 95 | 93 | 96.9 | 88.9 |
| 12:1 | 8 | 95 | 94.5 | 97.6 | 89.0 |
| 18:1 | 6 | 91 | 95 | 97.7 | 91.9 |

*Recoveries of the aromatic and paraffinic hydrocarbons are based upon the weight recovered as compared to the weight present in the primary feed.

**Purities are computed as a weight percent.

30.

## CLAIMS

1. A process for the dearomatization of a mixed hydrocarbon feed characterised by the following steps:

(a) contacting the mixed feed in an extraction zone with an aromatic selective solvent at a temperature of at least about 150°C to provide an aromatic-rich solvent phase containing primarily aromatic hydrocarbons and a raffinate containing primarily nonaromatic hydrocarbons;

(b) cooling the aromatic-rich solvent phase to bring about the formation of two phases;

(c) introducing said cooled aromatic-rich solvent phase of step (b) to a decantation zone to provide an aromatic-rich extract phase containing primarily aromatic hydrocarbons and a solvent phase containing primarily aromatic-selective solvent;

(d) recycling said solvent phase to the extraction zone of step (a); and

(e) recovering the aromatic-rich extract phase of step (c) and the raffinate of step (a).

2. A process as claimed in claim 1, characterised in that the solvent is a polyalkylene glycol.

3. A process as claimed in claim 2, characterised in that the solvent is diethylene glycol.

4. A process as claimed in any one of the preceding claims, characterised in that the temperature in the extraction zone is from about 150°C to about 250°C.

5. A process as claimed in any one of the preceding claims, characterised in that the temperature in the decantation zone is from about 75°C to about 125°C.

6. A process as claimed in any one of the preceding claims, characterised in that the ratio of solvent to feed in the extraction zone of step (a) is in the range of from about 4 to about 12 parts by weight of solvent to one part by weight of feed.

7. A process as claimed in any one of the preceding claims, characterised by the following additional steps:

(i) prior to step (d) contacting said solvent phase with at least a portion of said mixed feed in an extraction zone to provide a solvent phase containing primarily aromatic selective solvent and a secondary feed containing primarily aromatic and nonaromatic hydrocarbons; and

(ii) recycling said feed of (i) to the extraction zone of step (a).

8. A process as claimed in any one of claims 1 to 6, characterised by the following additional steps:

(i) prior to step (d) contacting said solvent phase with at least a portion of said raffinate of step (a) in an extraction zone to provide a solvent phase containing primarily aromatic selective solvent and a secondary raffinate containing primarily aromatic and nonaromatic hydrocarbons; and

(ii) introducing said secondary raffinate of (i) to the extraction zone of step (a).

9. A process as claimed in claim 7 or 8, characterised in that the temperature in the primary extraction zone is from about 150°C to about 250°C, the temperature in the decantation zone is from about 75°C to about 100°C; and the temperature in the secondary extraction zone is from about 50°C to about 125°C.

10. A process as claimed in claim 7 or 9, characterised in that the ratio of solvent to feed in the extraction zone of step (a) is in the range of about 4 to 12 parts by weight of solvent to one part by weight

32.

of feed and the ratio of solvent to raffinate of step (a) in the extraction zone of step (i) is in the range of about 10 to about 20 parts by weight of solvent to one part by weight of feed.

11. A process as claimed in claim 8 or 9, characterised in that the ratio of solvent to feed in the extraction zone of step (a) is in the range of about 2 to about 12 parts by weight of solvent to one part by weight of feed and the ratio of solvent to raffinate in the extraction zone of step (i) is in the range of about 10 to about 20 parts by weight of solvent to one part by weight of raffinate.

12. A process as claimed in any one of the preceding claims, characterised by the following additional steps:

(i) contacting the raffinate of step (a) and the aromatic-rich extract of step (c) with water to form a water phase containing primarily water and solvent;

(ii) extracting the water phase of step (i) with at least a portion of said mixed feed of step (a) at a temperature of from about 125°C to about 225°C to form a water phase containing primarily water and a feed containing primarily aromatic and nonaromatic hydrocarbons and small amounts of aromatic selective solvent;

(iii) recycling said water phase to step (i) and said feed to step (a).

13. A process as claimed in any one of claims 7 to 11, characterised by the following additional steps:

(iii) extracting the raffinate of step (a) and the aromatic-rich extract of step (c) with water to form a wash phase containing primarily water and solvent;

(iv) contacting the wash phase of step (iii) with at least a portion of said mixed feed of step (a) at a

33.

temperature of from about 125°C to about 225°C to form a water phase containing primarily water and a feed containing primarily aromatic and nonaromatic hydrocarbons and minor amounts of aromatic selective solvent.

(v)   recycling said water phase to step (iii) and said feed to step (a).

14.   Product of a process as claimed in any one of the preceding claims.

FIG. 1

FIG. 2

# FIG. 3

# FIG. 4

European Patent Office

**EUROPEAN SEARCH REPORT**

Application number

EP 81 30 2927

| | DOCUMENTS CONSIDERED TO BE RELEVANT | | CLASSIFICATION OF THE APPLICATION (Int Cl.³) |
|---|---|---|---|
| Category | Citation of document with indication, where appropriate, of relevant passages | Relevant to claim | |
| | GB - A - 865 698 (SHELL)<br>  * Page 2, lines 3-114; claims 1-14; figure *<br>  -- | 1-4,6 | C 10 G 21/00<br>21/28 |
| | US - A - 2 928 788 (J.L. JEZL)<br>  * Column 1, line 50 - column 2, line 61; figure 1 *<br>  -- | 1,3,8 | |
| | US - A - 3 725 257 (C. CAVENAGHI et al.)<br>  * Abstract; column 3, line 25 - column 4, line 20; column 5, lines 21-55; claims 1-11; figure *<br>  -- | 1,6 | TECHNICAL FIELDS SEARCHED (Int. Cl.³)<br><br>C 10 G 21/00<br>21/02<br>21/28 |
| | US - A - 2 161 567 (W.P. GEE)<br>  * Page 1, right-hand column, line 56 - page 2, right-hand column, line 13; claims 1-3; figure *<br>  -- | 1,6,7,9 | |
| | GB - A - 671 334 (TEXACO)<br>  * Page 1, lines 21-86; page 2, lines 6-18; page 3, lines 3-16; 19-95; claims 1-13; figure *<br>  -- | 1,12,13 | CATEGORY OF CITED DOCUMENTS<br>X: particularly relevant<br>A: technological background<br>O: non-written disclosure<br>P: intermediate document<br>T: theory or principle underlying the invention |
| D | US - A - 2 400 802 (G.B. ARNOLD)<br>  * Page 1, left-hand column, line 42 - page 2, right-hand column, line 48; claims 1-6 *<br>  --   ./. | 1-4,6 | E: conflicting application<br>D: document cited in the application<br>L: citation for other reasons<br><br>&: member of the same patent family, corresponding document |

The present search report has been drawn up for all claims

| Place of search | Date of completion of the search | Examiner |
|---|---|---|
| The Hague | 13-10-1981 | LO CONTE |

EPO Form 1503 1 06.78

| | European Patent Office | **EUROPEAN SEARCH REPORT** | Application number EP 81 30 2927 -2- |

## DOCUMENTS CONSIDERED TO BE RELEVANT

| Category | Citation of document with indication, where appropriate, of relevant passages | Relevant to claim |
|---|---|---|
| DA | US - A - 3 291 728 (A.A. BOYUM). | |
| DA | US - A - 1 908 018 (L.M. HENDERSON) | |
| A | US - A - 3 306 849 (P.P. BOZEMANN) | |

----

**CLASSIFICATION OF THE APPLICATION (Int. Cl.3)**

**TECHNICAL FIELDS SEARCHED (Int. Cl.3)**

EPO Form 1503.2   06.78